(19) Europäisches Patentamt
European Patent Office
Office européen des brevets

(11) Veröffentlichungsnummer: **0 007 415**
**B1**

(12) # EUROPÄISCHE PATENTSCHRIFT

(45) Veröffentlichungstag der Patentschrift: **05.08.81**

(51) Int. Cl.$^3$: **C 07 D 263/44**

(21) Anmeldenummer: **79101896.3**

(22) Anmeldetag: **11.06.79**

(54) **Verfahren zur Herstellung von Oxazoliden-2,4-dionen.**

(30) Priorität: **22.06.78 DE 2827414**

(43) Veröffentlichungstag der Anmeldung: **06.02.80 Patentblatt 80/3**

(45) Bekanntmachung des Hinweises auf die Patenterteilung: **05.08.81 Patentblatt 81/31**

(84) Benannte Vertragsstaaten: **AT BE CH DE FR GB IT LU NL SE**

(56) Entgegenhaltungen:
**DE-A-1 545 911**
**DE-A-2 002 410**
**DE-A-2 022 494**
**DE-A-2 207 576**
**DE-A-2 324 591**
**DE-A-2 711 659**
**DE-B-1 811 843**
**DE-C-729 852**

(73) Patentinhaber: **BASF Aktiengesellschaft, Carl-Bosch-Strasse 38, D-6700 Ludwigshafen (DE)**

(72) Erfinder: **Scholz, Herbert, Dr., Londoner Ring 7, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Verfahren zur Herstellung von Oxazolidin-2,4-dionen

Die Erfindung betrifft ein neues Verfahren zur Herstellung von Oxazolidin-2,4-dionen durch Umsetzung von Harnstoffen mit 2-Hydroxycarbonsäureestern.

Es ist bekannt, Oxazolidin-2,4-dione durch Umsetzung von Isocyanaten mit 2-Hydroxycarbonsäureestern herzustellen (DT-AS 1811843, DT-OS 2022494, DT-OS 2207576). Weitere Oxazolidin-dione sind aus der DE-OS 2002410 bekannt. Es ist ferner bekannt, Harnstoff mit Estern aliphatischer Oxysäuren zu 2,4-Dioxooxazolidin umzusetzen (DE-PS 729852). Hierbei wird überschüssiger Harnstoff mit salpetriger Säure oder Oxalsäure aus der Reaktionsmischung entfernt. Dies ist umständlich. Es ist daher vorteilhaft, eine Umsetzung durchzuführen, bei der dieser Verfahrensschritt nicht notwendig ist.

Es ist ferner bekannt, Harnstoff mit einem Ester einer alpha-Hydroxycarbonsäure in Gegenwart von Natriumäthylatherzustellen (DE-OS 1545911). Das Arbeiten mit Natriumäthylat ist umständlich und gefährlich, weil mit metallischem Natrium unter wasserfreien Bedingungen gearbeitet werden muss, um das Natriumäthylat herzustellen. Es ist daher vorteilhaft, eine Umsetzung durchzuführen, bei der in Abwesenheit von Alkalialkoholat gearbeitet wird.

Es wurde nun gefunden, dass man ein Oxazolidin-2,4-dion der Formel

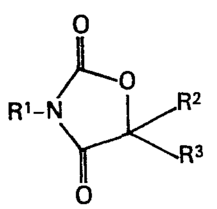

in der $R^1$ einen gegebenenfalls durch ein oder mehrere Halogenatome, Methyl oder Methoxy substituierter Arylrest mit 6 bis 12 Kohlenstoffatomen,

$R^2$ Wasserstoff oder einen Alkylrest mit 1 bis 4 C-Atomen oder einen Methoxymethylrest oder einen Vinylrest bedeutet und $R^3$ die gleichen Bedeutungen wie $R^2$ hat, wobei $R^2$ und $R^3$ im Einzelfall gleich oder verschieden sind, in Abwesenheit von Alkalialkoholat und ohne Verwendung von salpetriger Säure oder Oxalsäure in guter Ausbeute erhält, wenn man ein Harnstoffderivat der Formel

$R^1$ - NH - CO - $NHR^4$

in der $R^1$ die oben genannte Bedeutung hat und $R^4$ die gleiche Bedeutung wie $R^1$ hat oder Wasserstoff bedeutet, wobei $R^1$ und $R^4$ im Einzelfall gleich oder verschieden sind bei einer Temperatur zwischen 100 und 250 °C mit einem 2-Hydroxycarbonsäureester der Formel

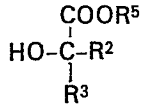

umsetzt, in der $R^5$ einen Alkylrest mit 1 bis 10 C-Atomen oder einen Cyclohexylrest bedeutet und $R^2$ und $R^3$ die oben genannten Bedeutungen haben.

Die verschiedenen Substituenten haben beispielsweise die folgenden Bedeutungen: $R^1$ und $R^4$ z.B. Phenyl, Naphthyl, 2-, 3- und 4-Fluorphenyl, 2-, 3- und 4-Chlorphenyl, 2-, 3- und 4-Bromphenyl, 2,3-Dichlorphenyl, 2,3,6-Trichlorphenyl, 2-, 3- und 4-Methoxyphenyl, 2-, 3- und 4-Methylphenyl, 3,4-Dichlorphenyl, 2,6-Dichlorphenyl, 2,3,4-Trichlorphenyl, 3,4,5-Trichlorphenyl, 2,4,6-Trichlorphenyl, 2,3,5-Trichlorphenyl und bevorzugt 3,5-Dichlorphenyl.

$R^4$ kann auch Wasserstoff bedeuten.

$R^2$ und $R^3$ z.B. Wasserstoff oder Vinyl oder Alkyl z.B. Methyl, Äthyl oder Methoxymethyl.

$R^5$ z.B. Methyl, Äthyl, Butyl, Isobutyl, Isooctyl, Nonyl, Decyl. Bevorzugte Reste sind $R^1$ und $R^4$ = 3,5-Dichlorphenyl,

$R^5$ = Isobutyl, Methyl, Butyl, $R^2$ = Methyl und $R^3$ = Vinyl.

Für die Durchführung der Umsetzung ist eine erhöhte Temperatur zwischen 100 und 250 °C, vorzugsweise zwischen 150 und 240 °C erforderlich.

Die Umsetzung kann auch in Gegenwart eines Trialkylamins z.B. Tributylamin durchgeführt werden. Hierdurch wird die Umsetzungstemperatur erniedrigt.

Die Umsetzung kann ohne Lösungsmittel durchgeführt werden. Lösungsmittel mit einem Siedepunkt, der im oben genannten Temperaturbereich liegt, können verwendet werden.

Für die Umsetzung werden vorteilhaft stöchiometrische Mengen der Ausgangsprodukte verwendet. Es ist jedoch auch möglich einen Überschuss, beispielsweise bis zu 10%, an einem der Ausgangsprodukte, zweckmässig an dem billigeren Ausgangsprodukt, zu verwenden.

Wichtig für eine gute Ausbeute bei der Umsetzung ist es, die entstehenden Amine $R^4$ NH2 oder $R^1$ NH2 und den entstehenden Alkohol $R^5$ OH möglichst quantitativ abzutrennen, vorzugsweise abzudestillieren und dafür zu sorgen, dass dabei der als Ausgangsprodukt verwendete 2-Hydroxycarbonsäureester nicht mitdestilliert. Zu diesem Zweck ist die Verwendung von Destillationskolonnen mit Füllkörpern besonders vorteilhaft.

Die Umsetzung kann bei Normaldruck durchgeführt werden; ein Vakuum von 5 bis 950 mbar kann von Vorteil sein, um die Destillationstemperatur zu erniedrigen.

Die als Ausgangsprodukte verwendeten Harnstoffderivate erhält man z.B. durch Umsetzung eines Isocyanates mit einem Amin in üblicher Weise. So erhält man beispielsweise durch Umsetzung von 3,5-Dichlorphenylisocyanat mit 3,5-Dichloranilin den Bis-(3,5-dichlorphenyl)-harnstoff (Fp 294 °C). Demgegenüber ist es überraschend, dass das erfindungsgemässe Verfahren in einfacher Weise abläuft und die Endprodukte in guter Ausbeute erhalten werden.

Die folgenden Beispiele erläutern die Durchführung des erfindungsgemässen Verfahrens.

Beispiel 1

Eine Suspension von 210 Teilen (Gewichtsteilen) (0,6 mol) Bis-(3,5-dichlorphenyl)-harnstoff und 105,3 Teilen (0,6 mol) Vinylmilchsäureisobutylester werden 15 Minuten unter Rückfluss erhitzt. Über eine 40 cm hohe Destillationskolonne (mit 2 cm Durchmesser, gefüllt mit Maschendrahtringen mit 2400 Maschen/$cm^2$ aus nichtrostendem Stahl) werden bei Normaldruck bei einer Temperatur der Mischung von 220 bis 232 °C und einer Dampftemperatur von 103 bis 106,5 °C 19 Teile (Volumenteile) Isobutanol abdestilliert. Anschliessend werden bei einem Druck von 200 bis 170 mbar und einer Temperatur der Mischung von 205 bis 215 °C und einer Dampftemperatur von 68,5–64,5 °C weitere 25 Volumenteile Isobutanol abdestilliert. Der Rückstand wird auf 80 °C abgekühlt; dazu gibt man 150 Teile Methanol. Man rührt 2 Stunden bei 20 °C nach, saugt das abgeschiedene Festprodukt ab und wäscht zweimal mit je 20 Teilen Methanol.

Ausbeute: 145,6 Teile 3-(3,5-Dichlorphenyl)-5-methyl-5-
vinyl-1,3-oxazolidin-2,4-dion
(= 85% Ausbeute)
Fp: 101–104 °C

Beispiel 2

Eine Suspension von 175 Teilen (0,5 mol) Bis-(3,5-dichlorphenyl)-harnstoff in 66,1 Teilen (0,5 mol) 2-Hydroxyisobuttersäureäthylester und 9,3 Teilen (0,5 mol) Tributylamin wird 1 Stunde unter Rückfluss erhitzt. Anschliessend destilliert man unter Normaldruck bei einer Temperatur der Mischung von 150 bis 230 °C und einer Dampftemperatur von 77 bis 80 °C über eine Destillationskolonne (Daten wie im Beispiel 1) 18 Volumenteile Äthanol ab.

Der Rückstand wird auf 50 °C abgekühlt und mit 150 Teilen Methanol versetzt. Man rührt 2 Stunden bei 20 °C nach, saugt das abgeschiedene Festprodukt ab und wäscht zweimal mit je 20 Teilen Methanol.

Ausbeute: 120,1 Teile 3-(3,5-Dichlorphenyl)-5,5-dimethyl-
1,3-oxazolidin-2,4-dion
(= 87,6% Ausbeute)
Fp: 166–167 °C

Beispiel 3

Eine Suspension aus 90 Teilen (0,5 mol) 98% Vinylmilchsäureisobutylester und 102,5 Teilen (0,5 mol) N-(3,5-Dichlorphenyl)-harnstoff (Fp 190 °C, beispielsweise durch Umsetzung von 3,5-Dichlorphenylisocyanat mit Ammoniak herstellbar) wird mit 11 Teilen (0,05 mol) Tributylamin eine halbe Stunde unter Rückfluss erhitzt. Bei 150 mbar Druck und einer Temperatur der Mischung von 160–201 °C und einer Dampftemperatur von 68 bis 86 °C werden 32 Volumenteile Destillat erhalten. Der Rückstand wird auf 80 °C abgekühlt; dazu gibt man 100 Teile Methanol. Man rührt 2 Stunden bei 20 °C nach, saugt das abgeschiedene Festprodukt ab und wäscht zweimal mit je 20 Teilen Methanol.

Mit einer etwas geringeren Ausbeute als im Beispiel 1 beschrieben erhält man 3-(3,5-Dichlorphenyl)-5-methyl-5-vinyl-1,3-oxazolidin-2,4-dion Fp: 107–109 °C.

**Patentansprüche**

1. Verfahren zur Herstellung von Oxazolidin-2,4-dionen der Formel

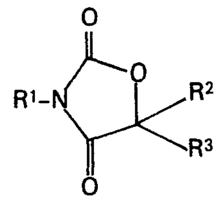

in der $R^1$ einen gegebenenfalls durch ein oder mehrere Halogenatome, Methyl oder Methoxy substituierter Arylrest mit 6 bis 12 Kohlenstoffatomen,

$R^2$ Wasserstoff oder einen Alkylrest mit 1 bis 4 C-Atomen oder einen Methoxymethylrest oder einen Vinylrest bedeutet und $R^3$ die gleichen Bedeutungen wie $R^2$ hat, wobei $R^2$ und $R^3$ im Einzelfall gleich oder verschieden sind, dadurch gekennzeichnet, dass man ein Harnstoffderivat der Formel

$$R^1 - NH - CO - NH - R^4$$

in der $R^1$ die oben genannten Bedeutungen hat und $R^4$ die gleiche Bedeutung wie $R^1$ hat oder Wasserstoff bedeutet, wobei $R^1$ und $R^4$ im Einzelfall gleich oder verschieden sind bei einer Temperatur zwischen 100 und 250 °C mit einem 2-Hydroxycarbonsäureester der Formel

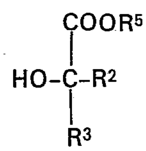

umsetzt, in der $R^5$ einen Alkylrest mit 1 bis 10 C-Atomen oder einen Cyclohexylrest bedeutet.

2. Verfahren gemäss Anspruch 1, dadurch gekennzeichnet, dass man die bei der Umsetzung entstehenden Amine $R^4NH_2$ oder $R^1NH_2$ und den entstehenden Alkohol $R^5OH$ abtrennt, vorzugsweise abdestilliert.

3. Verfahren gemäss Anspruch 1, dadurch gekennzeichnet, dass man die Umsetzungen mit Verbindungen durchführt, bei denen $R^1$ und $R^4$ 3,5-Dichlorphenyl, $R^2$ Methyl und $R^3$ Vinyl bedeuten.

**Claims**

1. A process for the preparation of an oxazolidine-2,4-dione of the formula

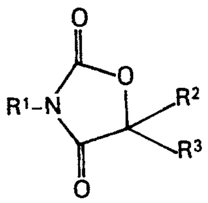

where $R^1$ is aryl of 6 to 12 carbon atoms which is unsubstituted or substituted by one or more halogen atoms, methyl or methoxy, $R^2$ is hydrogen, alkyl of 1 to 4 carbon atoms, methoxymethyl or vinyl, and $R^3$ has the same meanings as $R^2$, and in any particular compound $R^2$ and $R^3$ may be identical or different, characterized in that a urea derivative of the formula

$$R^1 - NH - CO - NH - R^4$$

where $R^1$ has the above meanings and $R^4$ has the same meanings as $R^1$ or is hydrogen, and in any particular compound $R^1$ and $R^4$ may be identical or different, is reacted with a 2-hydroxycarboxylic acid ester of the formula

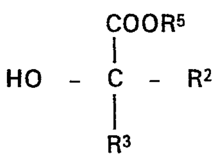

where $R^5$ is alkyl of 1 to 10 carbon atoms or cyclohexyl, at a temperature of from 100 to 250 °C.

2. A process as claimed in claim 1, characterized in that the amines $R^4NH_2$ or $R^1NH_2$ formed in the reaction and the alcohol $R^5OH$ formed are removed, preferably by distillation.

3. A process as claimed in claim 1, characterized in that the reaction is carried out with a compound in which $R^1$ and $R^4$ denote 3,5-dichlorophenyl, $R^2$ denotes methyl and $R^3$ denotes vinyl.

**Revendications**

1. Procédé de préparation de dérivés d'oxazolidine-2,4-dione de la formule

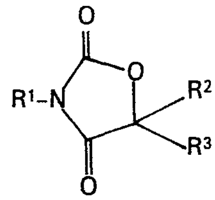

dans laquelle
$R^1$ représente un groupe aryle en $C_6$ à $C_{12}$, éventuellement substitué par un ou plusieurs atomes d'halogène ou des radicaux méthyle ou méthoxy,
$R^2$ désigne un atome d'hydrogène ou un radical alcoyle en $C_1$ à $C_4$ ou un groupe méthoxy-méthyle ou vinyle,
$R^3$ possède les significations définies pour $R^2$, $R^2$ et $R^3$ pouvant être identiques ou différents,
caractérisé en ce que l'on fait réagir un dérivé d'urée de la formule

$$R^1 - NH - CO - NH - R^4,$$

dans laquelle $R^1$ possède les significations définies et $R^4$ possède les mêmes significations que $R^1$, mais peut en outre représenter un atome d'hydrogène, $R^1$ et $R^4$ pouvant être identiques ou différents,
à une température comprise entre 100 et 250 °C avec un ester d'acide 2-hydroxy-carboxylique de la formule

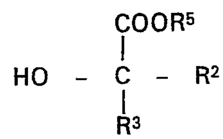

dans laquelle $R^5$ représente un radical alcoyle en $C_1$ à $C_{10}$ ou un groupe cyclohexyle.

2. Procédé suivant la revendication 1, caractérisé en ce que les amines $R^4$-$NH_2$ ou $R^1$-$NH_2$ et l'alcool $R^5$-OH formés pendant la réaction sont séparés, de préférence par distillation.

3. Procédé suivant la revendication 1, caractérisé en ce que la réaction est réalisée avec des composés de départ, pour lesquels $R^1$ et $R^4$ désignent des groupes 3,5-dichloro-phényle, $R^2$ est un radical méthyle et $R^3$ un groupe vinyle.